# FASCICULE DE BREVET EUROPEEN

(11) **EP 3 391 926 B1**
(45) Date de publication et mention de la délivrance du brevet: **30.10.2019**
(21) Numéro de dépôt: 18166932.6
(22) Date de dépôt: 12.04.2018
(51) Int. Cl.: A61M 16/10, A61M 39/20, A61M 39/22, F17D 5/02, A61M 16/08, F16L 37/60, F16K 15/02, A61M 39/26, A61M 39/24, A61M 39/10, A61M 16/00, A61M 16/20, F16L 37/40, F16K 17/04, G16H 40/67, G16H 20/40, F16K 37/00, F16K 15/03

(54) **PRISE DE DISTRIBUTION DE GAZ CONNECTÉE À DÉBITMÈTRE INTÉGRÉ**
GASAUSLASSANSCHLUSS, DER MIT EINEM INTEGRIERTEN DURCHFLUSSMESSER VERBUNDEN IST
GAS DISTRIBUTION SOCKET CONNECTED TO AN INTEGRATED FLOWMETER

(30) Priorité: 19.04.2017 FR 1753382
(43) Date de publication de la demande: 24.10.2018
(73) Titulaire: L'AIR LIQUIDE, SOCIETE ANONYME POUR L'ETUDE ET L'EXPLOITATION DES PROCEDES GEORGES CLAUDE, 75007 Paris (FR); AIR LIQUIDE SANTE (INTERNATIONAL), 75007 Paris (FR)
(72) Inventeur: DUDRET, Stéphane, 75015 Paris (FR); PIGASSOU, Jeanne, 92200 Neuilly sur Seine (FR); BOEUF, Franck, 78110 Le Vesinet (FR)
(74) Mandataire: Pittis, Olivier

(56) Documents cités:
- EP-A1- 2 682 150
- EP-A1- 3 369 452
- EP-A2- 1 983 251
- US-A1- 2013 317 379

## Description

L'invention concerne une prise de distribution de gaz connectée ou communicante, en particulier de gaz médical, conçue pour être fixée sur une paroi d'un bâtiment hospitalier ou analogue, laquelle est équipée d'un débitmètre à oscillation fluidique et de moyens de télécommunication, et son utilisation pour distribuer un gaz au sein d'un bâtiment hospitalier, en particulier de l'oxygène, de l'air ou du protoxyde d'azote (N₂O).

Dans un hôpital ou analogue, les gaz respiratoires qui sont administrés aux patients sont acheminés jusque dans les salles de soins, les chambres ou autre, via un réseau de canalisations de gaz traversant le bâtiment hospitalier.

La connectique entre le réseau de canalisations de gaz et le patient est assurée grâce à plusieurs composants, notamment un ou des tuyaux flexibles, une interface patient, tel un masque respiratoire, des canules nasales ou une sonde trachéale par exemple, éventuellement un appareil d'assistance respiratoire ou analogue, et une connectique appelée « prise » ou «embout» de raccordement et de distribution de fluides médicaux, comme décrit par exemple par FR-A-2899950, US-A-3532101 ou EP-A-2306060.

Le document EP 1 983 251 A2 divulgue une prise communicante de distribution de gaz.

Le document EP 3 369 452 A1 est uniquemenet état de la technique selon l'article 54(3) CBE et divulgue un appareil de traitement médical à débitmètre à oscillation fluidique et module de communication longue distance.

Les prises de distribution de fluide sont habituellement fixées sur les paroi ou murs des bâtiments hospitaliers et sont conçues pour connecter fluidiquement le réseau de canalisations de gaz à un conduit flexible alimentant le patient en gaz respiratoire, ledit conduit étant équipé à son extrémité de connexion d'un embout de fixation complémentaire de la prise.

Une telle prise de distribution de fluide intègre typiquement un passage de gaz muni d'une vanne, qui est maintenue fermée si un conduit ou un autre appareil n'est pas connecté à la prise de distribution de fluide, et un filtre servant à purifier le gaz destiné au patient en le débarrassant des impuretés éventuelles, notamment des poussières, microorganismes....

En général, un sélecteur de débit ou débitlitre est fixé en aval de cette prise murale par rapport au patient afin de contrôler le débit de gaz envoyé au patient. Le débitlitre sert à ajuster le débit et éventuellement la pression du fluide, i.e. du gaz, et affiche la valeur de débit sélectionnée.

Le sélecteur de débit ou débitlitre peut être du type à orifices calibrés et réducteur de pression intégré, comme le dispositif Selectaflo™ d'Air Liquide Medical Systems, ou du type à bille.

Or, la valeur exacte du débit délivré au patient n'est pas connue précisément. En effet, les sélecteurs de débit ou débitlitres connus donnent une valeur approximative du débit, avec une précision:
- de ± 30% de la valeur lue jusqu'à 1,5 L/min et de ± 20% de la valeur lue au-delà de 1,5 L/min pour un débitlitre à détendeur intégré, et
- de +/- 10% de la valeur lue ou +/- 0,5 L/min (valeur la + élevée à 23°C) pour le débitlitre à bille.

Par ailleurs, un sélecteur de débit ne contient pas d'électronique et ne communique donc pas à distance la ou les valeurs de débit sélectionnées par le personnel soignant.

Le problème qui se pose dans ce contexte est de pouvoir suivre à distance et aussi précisément que possible, et de préférence en permanence, le débit de gaz distribué par une ou plusieurs prises de distribution de fluide, en particulier une ou des prises murales équipant un bâtiment hospitalier ou analogue de manière à pouvoir déterminer la quantité de gaz délivrée à un ou des patients soignés au sein dudit bâtiment hospitalier. De préférence, le suivi du débit doit pouvoir être assuré sur une longue période, par exemple plusieurs années et ce, sans nécessité d'opérations de maintenance trop fréquentes.

La solution de l'invention concerne alors une prise de distribution de gaz communicante, selon la revendication 1.

Selon le cas, la prise de distribution de gaz de l'invention peut comprendre l'une ou plusieurs des caractéristiques techniques suivantes :
- le passage de gaz interne comprend une extrémité libre formant embout de connexion pour le raccordement d'un connecteur, par exemple un connecteur de raccordement équipant un tuyau flexible servant à convoyer du gaz.
- le passage de gaz interne comprend un orifice d'entrée de gaz et un orifice de sortie de gaz.
- le système de contrôle du débit de gaz comprend des moyens à vanne autorisant une circulation du gaz dans le passage de gaz interne du corps de prise, en direction d'un orifice de sortie de gaz porté par l'extrémité libre formant embout de connexion, lorsqu'un connecteur est raccordé à ladite l'extrémité libre formant embout de connexion.
- le débitmètre à oscillation fluidique est agencé dans le corps de prise.
- les moyens de traitement de signal sont reliés électriquement au débitmètre à oscillation fluidique.
- ledit au moins un module de télécommunication est relié électriquement aux moyens de traitement de signal et/ou configuré pour transmettre sans fil un ou des signaux de mesure provenant des moyens de traitement de signal à au moins un réseau de communication.
- les moyens à vanne comprennent un clapet et un siège clapet.
- ledit au moins un module de télécommunication est configuré pour communiquer des données selon une technologie de type LoRa, Sigfox, LTE-M Cat 0, EC-GSM ou NB-loT.
- elle est configurée, c'est-à-dire conçue ou adaptée, pour être fixée à ou encastrée dans une structure-support, par exemple une paroi ou un mur.
- elle comprend en outre une source de courant électrique alimentant les moyens de traitement de signal et/ou le module de télécommunication.
- les moyens de traitement de signal comprennent une carte électronique, encore appelée électronique embarquée.
- le module de télécommunication est configuré pour transmettre sans fil au moins une valeur de débit provenant des moyens de traitement de signal à au moins un réseau de communication.
- les moyens à vanne agencés sur le passage de gaz interne du corps de prise comprennent un clapet coopérant avec un siège de clapet.
- elle comprend un élément d'obturation mobile permettant de recouvrir au moins une partie de la face proximale du corps de prise comportant l'orifice de sortie du passage interne de gaz.
- l'élément d'obturation mobile comprend un capot ou clapet monté sur charnière.
- une antenne est agencée sur l'élément d'obturation mobile.
- alternativement, une antenne est agencée sur la face proximale de la prise, à l'intérieur ou à l'extérieur du corps de prise ou en étant incorporé à la structure-même du corps de prise.
- alternativement, une antenne est agencée sur dans une portion externe de la prise qui est elle-même encastrée dans une structure-support de type cloison, paroi, mur ou analogue, de préférence structure-support agencée (quasi) verticalement par rapport au sol.
- les moyens de traitement de signal comprennent au moins un premier module de télécommunication.
- les moyens de traitement de signal comprennent au moins un premier modem radio, une première antenne radio et un premier réseau d'adaptation d'impédance.
- les moyens de traitement de signal sont configurés pour communiquer des données selon une technologie de type LoRa, Sigfox, LTE-M Cat 0, EC-GSM ou NB-IoT.
- le premier module de télécommunication présente avantageusement une sensibilité inférieure ou égale à -110 dBm et une valeur d'affaiblissement de couplage ou valeur « MCL » supérieure ou égale à 130 dB avec la ou les stations de base du réseau de communication considéré de manière à pouvoir réaliser des transmissions de données sur de longues distances, typiquement de 100 m à 30 km environ, selon l'environnement de propagation des signaux considérés, i.e. milieu urbain ou dégagé.
- le premier modem radio est configuré pour opérer au moins une modulation du type à étalement de spectre, de préférence au moins une modulation de type LoRa, éventuellement associée à un protocole d'accès de type LoRaWAN ; ou du type à bande ultra-étroite, de préférence au moins une modulation de type Sigfox, éventuellement associée à un protocole d'accès Sigfox.
- alternativement, le premier modem radio est configuré pour opérer au moins une modulation et un protocole ou une pile de protocoles associés, sur au moins à un réseau de type EC-GSM, NB-loT ou LTE-M.
- le premier modem radio est configuré pour opérer selon un débit de transmission compris entre 100 bits par seconde, comme dans le cas d'un réseau Sigfox, et 1 Mégabit par seconde, comme dans le cas d'un réseau LTE-M Cat0, c'est-à-dire dans tout ou partie de cette plage de débit de transmission.
- les moyens de traitement de signal comprennent un second module de télécommunication comprenant un deuxième modem radio, une deuxième antenne radio et un deuxième réseau d'adaptation d'impédance configuré pour communiquer des données selon une seconde technologie de type Bluetooth Low Energy, Bluetooth ou NFC.
- les moyens de traitement de signal comprennent un premier et un second module de télécommunication et une antenne radio unique commune aux deux modules de télécommunication.
- alternativement, les moyens de traitement de signal comprennent un premier et un second module de télécommunication et une antenne radio unique dissociée en deux sous-antennes distinctes spécifiques chacune d'une technologie radio, donc associées chacune à l'un des modules de télécommunication.
- les moyens de traitement de signal comprennent en outre une ou plusieurs mémoires électroniques et/ou des moyens analogiques ou numériques de conditionnement des signaux générés par le moyen de détection des oscillations fluidiques du débitmètre à oscillateur fluidique.
- ledit au moins un réseau de communication comprend ou est relié à un serveur ou un ordinateur distant.
- elle comprend un capteur de position permettant de déterminer la position « ouverte » ou « fermée » de l'élément d'obturation mobile.
- le capteur de position est agencé au niveau de la face avant de la prise.
- le capteur de position est un capteur capacitif ou un contacteur REED réagissant au voisinage d'un élément de détection, tel un élément métallique ou un aimant, agencé sur l'élément d'obturation mobile.
- le capteur de position coopère avec l'élément de détection porté par l'obturateur pour déterminer la position « ouverte » ou « fermée » dudit obturateur.
- elle comprend une interface utilisateur reliée électriquement aux moyens de traitement de signal, c'est-à-dire une carte électronique ou analogue.
- elle comprend une interface utilisateur agencée en face avant ou proximale de la prise.
- l'interface utilisateur comprend un afficheur de données.
- l'interface utilisateur comprend un ou plusieurs témoins, notamment de fonctionnement et/ou de retour d'état de l'électronique embarquée, telles des diodes électroluminescentes.
- l'interface utilisateur comprend un ou plusieurs boutons, touches ou interrupteurs permettant par exemple de commander la marche ou l'arrêt de tout ou partie de l'électronique embarquée ou de l'interface utilisateur, la remise à zéro de la valeur intégrée du débit instantané, un changement de configuration de la prise, un envoi de données par radio, un appairage avec un dispositif tiers à travers une liaison radio Bluetooth ou Bluetooth Low Energy, ou de toute autre fonction.

D'une façon générale, la présente invention propose de combiner à la fonction de prise de distribution de gaz, une fonction de mesure de débit par incorporation au sein de ladite prise de distribution de gaz, un débitmètre par oscillateur fluidique, et de communiquer à distance les données de fréquence issues du débitmètre, après traitement de ces données de fréquence pour les transformer en données de débit, via un moyen de télécommunication sans fil, par exemple par BLE, WiFi ou tout autre système de télétransmission.

En d'autres termes, la prise de distribution de gaz de l'invention comprend donc :
- un conduit ou passage interne de gaz, de préférence en matière métallique, se terminant par un embout de raccordement et de distribution de gaz, ledit passage interne de gaz comprenant un clapet ou tout autre système d'obturation faisant office de vanne pour ouvrir le passage interne de gaz, lorsque l'on insère un connecteur adapté dans l'embout extérieur de la prise et, à l'inverse, fermer le passage interne de gaz, lorsque celui-ci est retiré.
- optionnellement un (ou plusieurs) filtre permettant d'éliminer tout ou partie des impuretés présentes dans le gaz. De préférence, le filtre est interchangeable, notamment après 1 ou plusieurs années de fonctionnement, par exemple tous les 2 ans.
- un débitmètre à oscillation fluidique, agencé sur le passage de gaz, permettant une mesure du débit de gaz par l'intermédiaire de deux microphones placés sur le trajet du gaz.
- de préférence, les deux microphones sont séparés de manière étanche du flux de gaz.
- le débitmètre à oscillation fluidique mesure une fréquence d'oscillation du flux, donc indirectement le débit de gaz pour des débits allant de 0.5 à 15 sL/min, avec une précision de 1% de la pleine échelle.
- des moyens de traitement de données, typiquement une carte électronique, aussi appelée « électronique embarquée », permettant le traitement des signaux de fréquence mesurées par le débitmètre pour les transformer en valeurs de débit et de transmettre ces données, i.e. les valeurs de débit, par télécommunication sans fil, par exemple à un ordinateur ou à un serveur distant.
- une source d'énergie, telle une (ou des) batterie ou pile ou une alimentation de type secteur (e.g., 10V/220V).
- de préférence, la source d'énergie comprend une (ou des) batterie ou pile.
- optionnellement, un élément d'obturation, tel un volet ou un capot pivotant, servant à cacher l'embout de la prise.
- un module de télécommunication avec un (ou des) modem et une (ou des) antenne agencée de préférence sur la face extérieure de la prise et en contact avec l'air ambiant. Ainsi, l'antenne peut être agencée sur le corps de prise ou sur l'élément d'obturation, tel un volet picotant.
- un boitier ou corps de prise incorporant les éléments précédents, comprenant un embout de sortie et de raccordement compatible avec les prises ou connecteurs de gaz utilisés à l'hôpital, i.e. lesquels ont des encoches et diamètres différents selon le gaz considéré, notamment l'oxygène, l'air ou le N₂O. L'embout de sortie est agencé à l'extrémité aval du conduit ou passage interne de gaz par laquelle sort le gaz utilisé.
- les deux microphones du débitmètre à oscillation fluidique conçus et agencés pour mesurer une fréquence d'oscillation de tourbillons de gaz.
- les moyens de traitement de signal sont reliés électriquement aux microphones du débitmètre à oscillation fluidique.
- les moyens de traitement de signal, reliés électriquement aux microphones, sont configurés pour déduire de la fréquence d'oscillation de tourbillons de gaz mesurée par lesdits microphones, un débit de gaz compris entre environ 0,5 et 15 sL/min (i.e., standard litre par minute).
- la source de courant électrique comprend au moins une pile ou batterie conçue pour assurer une fourniture de courant pendant plusieurs mois, de préférence au moins 1 an, avantageusement au moins 2 à 4 ans, préférentiellement plus de 3 ans, voire plus de 4 ans, ce qui permet notamment de réduire la fréquence des opérations de maintenance (i.e. changement de la pile) et/ou aussi de pouvoir implanter la prise dans des endroits difficiles, notamment sans alimentation électrique à proximité.

De façon générale, la prise de distribution de gaz selon l'invention permet de mesurer indirectement le débit de gaz distribué par la prise, notamment le gaz consommé par le patient situé en aval, et de transmettre cette information de débit à distance et sans fil, par exemple à un serveur distant. Elle combine donc les fonctions de vanne, de mesure de débit et de communication à distance des données, et éventuellement de filtrage du gaz.

La prise de distribution de gaz à débitmètre à oscillation fluidique selon l'invention présente de nombreux avantages par rapport à une prise incluant un débitmètre classique (i.e., non oscillant).

Ainsi, la prise selon l'invention à débitmètre à oscillation fluidique consomme nettement moins d'énergie électrique qu'une prise à débitmètre à fil chaud ou déprimogène, à savoir jusqu'à un rapport de 100 fois, ce qui permet, outre les économies d'énergie réalisées, de pouvoir alimenter la prise en courant électrique provenant d'une pile ou batterie, plutôt que du secteur électrique, donc de réduire l'encombrement global de la prise en augmentant sa compacité.

De plus, le fait de pouvoir y insérer une pile ou analogue pour assurer son alimentation électrique, permet de simplifier l'architecture générale de l'installation mais sans pénaliser son autonomie puisqu'un fonctionnement sur pile ou batterie peut être garanti jusqu'à plusieurs années dans ces conditions.

Une prise selon l'invention à débitmètre à oscillation fluidique est donc nettement plus performante qu'une prise à débitmètre classique.

L'invention porte en outre sur l'utilisation d'une prise de distribution de gaz communicante selon l'invention pour distribuer un gaz au sein d'un bâtiment hospitalier, en particulier de l'oxygène, de l'air ou du protoxyde d'azote (N₂O).

Par ailleurs, l'invention porte aussi sur un bâtiment hospitalier, par exemple un hôpital, une clinique ou analogue, comprenant au moins une prise de distribution de gaz communicante selon l'invention pour distribuer un gaz au sein dudit bâtiment hospitalier, en particulier de l'oxygène, de l'air ou du protoxyde d'azote (N₂O).

La (ou les) prise de distribution de gaz communicante selon l'invention sont agencées sur le réseau de canalisations de gaz parcourant le bâtiment hospitalier.

L'invention va maintenant être mieux comprise grâce à la description détaillée suivante, faite à titre illustratif mais non limitatif, en référence aux figures annexées parmi lesquelles :
- la Figure 1 est un schéma du principe de fonctionnement d'un débitmètre à oscillation fluidique équipant une prise murale de distribution de gaz selon l'invention,
- la Figure 2 est schéma tridimensionnel du débitmètre à oscillation fluidique de la Figure 1,
- les Figures 3 et 9 schématisent un premier mode de réalisation d'une prise de distribution de gaz en position « ouverte » selon la présente invention, laquelle intègre un débitmètre à oscillation fluidique selon les Figures 1 et 2,
- la Figure 4 illustre la prise de distribution de gaz de la Figure 3 en position « fermée »,
- la Figure 5 illustre un second mode de réalisation de la prise de distribution de gaz analogue à celui de la Figure 3, dans lequel une antenne est agencée sur la face proximale de la portion aval du boitier de la prise de distribution de gaz,
- la Figure 6 illustre un troisième mode de réalisation de la prise de distribution de gaz analogue à celui de la Figure 3, dans lequel une antenne est agencée dans la portion externe de la prise,
- la Figure 7 illustre un quatrième mode de réalisation de la prise de distribution de gaz analogue à celui de la Figure 3, dans lequel une antenne est agencée sur l'élément d'obturation mobile,
- la Figure 8 schématise l'agencement d'un capteur de position de l'élément d'obturation mobile sur la prise de la Figure 3, et
- la Figure 10 schématise un autre mode de réalisation de la prise de distribution de gaz des Figures 3 et 9 comprenant une interface utilisateur.

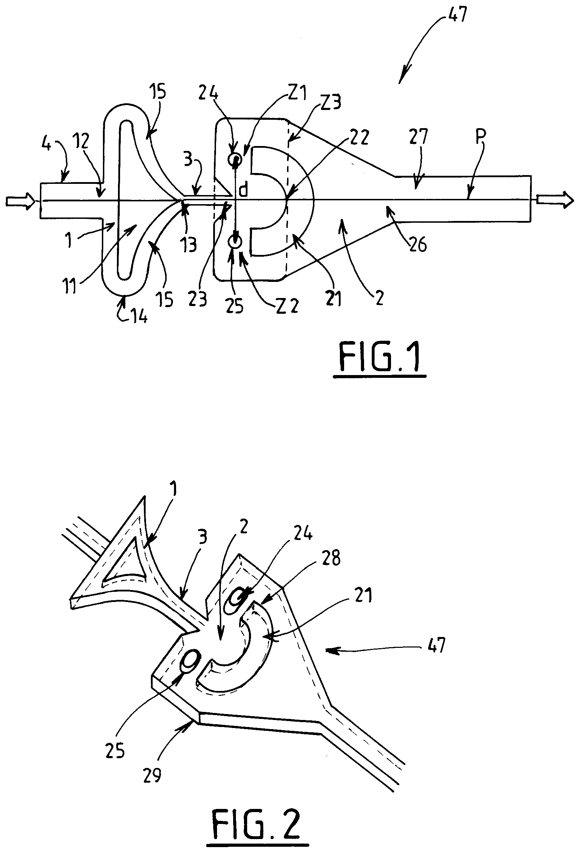

La Figure 1 est un schéma du principe de fonctionnement d'un débitmètre à oscillation fluidique 47 (vue de dessus) pour une prise de distribution de fluide 31, en particulier de gaz médical, selon l'invention, en particulier d'oxygène, de protoxyde d'azote (N₂O) ou d'air médical.

Plus précisément, le débitmètre à oscillation fluidique 47 comprend une chambre de stabilisation 1 dans laquelle est agencé un élément stabilisateur de flux 11, ayant ici une section générale sensiblement triangulaire ou quasi-triangulaire et une face avant, et une chambre d'oscillation 2 comprenant un élément à reflux 21 ayant ici une forme de demi-cylindre (i.e. une section hémicirculaire), lequel est configuré en arc de cercle 22 pour créer un tourbillon ou vortex gazeux oscillant. Le tourbillon oscille en fait entre deux zones Z1, Z2 situées schématiquement au niveau des extrémités du demi-cylindre de l'élément à reflux 21.

L'élément à reflux 21 est pris en sandwich entre deux parois parallèles 28, 29 délimitant la chambre d'oscillation 2 en haut et en bas respectivement (Figure 2), c'est-à-dire formant le plafond et le sol de la chambre d'oscillation 2.

Un conduit de liaison 3 relie fluidiquement la chambre de stabilisation 1 à la chambre d'oscillation 2 de sorte que le gaz qui entre dans la chambre de stabilisation 1, la traverse et alimente ensuite la chambre d'oscillation 2. Le conduit de liaison 3 y débouche en vis-à-vis, c'est-à-dire en face ou en regard, de l'élément à reflux 21 comprenant une cavité évidée de section semi-circulaire, i.e. préférentiellement hémicirculaire, ce qui engendre une oscillation du flux et une formation de tourbillons dans les deux zones Z1 et Z2 suscitées. Par exemple, l'élément à reflux 21 peut avoir une forme générale semi-cylindrique comme sur la Figure 1, ou une forme générale substantiellement ou quasi-semi-cylindrique.

Un plan de symétrie P sépare l'ensemble du système, en particulier le conduit de liaison 3, la chambre de stabilisation 1, l'élément stabilisateur de flux 11, la chambre d'oscillation fluidique 2 et l'élément à reflux 21, en deux parties égales et symétriques par rapport à ce plan de symétrie P.

La face avant de l'élément stabilisateur de flux 11 compris dans la chambre de stabilisation 1 est plate et perpendiculaire au plan de symétrie P, donc perpendiculaire à l'axe du conduit de liaison 3.

Une telle configuration est décrite dans la publication : Yves Le Guer; Jet confiné, dispersions fluide-particules et mélange chaotique; Engineering Sciences; Université de Pau et des Pays de l'Adour; 2005, et dans le document WO-A-93/22627.

Pour assurer une mesure efficace de la variation de la pression du gaz, en fonction du temps, au sein de la chambre à reflux 2 dans laquelle oscille le flux gazeux en formant des tourbillons gazeux dans les zones Z1, Z2, il convient de positionner les sites de mesure, c'est-à-dire les orifices de mesure 24, 25, reliés à des microphones ou à des capteurs de pression, de préférence des microphones (non représentés), dans le plafond (ou dans le sol) de la chambre à reflux 2, c'est-à-dire approximativement au-dessus des zones Z1, Z2 où se forment les tourbillons, et surtout symétriquement par rapport au plan de symétrie P du débitmètre en respectant impérativement entre eux, une distance d (mesurée entre les axes ou centres des orifices de mesures comprise entre environ 0,5 et 15 mm (cf. Figure 1), de préférence entre environ 0,5 et 10 mm, par exemple de l'ordre d'environ 1 à 6 mm.

Les deux orifices de mesure 24, 25 reliés de préférence à des microphones se situent de façon préférentielle sur un axe perpendiculaire au plan de symétrie P, et de façon préférentielle dans la zone Z3 représentée en pointillés sur la Figure 1. Le positionnement des deux orifices de mesure 24, 25, l'un par rapport à l'autre, ainsi que par rapport à d'autres éléments de la géométrie du système de débitmètre joue un rôle important dans la perception de la fréquence d'oscillation de la pression du tourbillon et par conséquent influence la précision de calcul du débit à partir des valeurs de pression mesurées par les capteurs reliés aux deux orifices de mesure 24, 25.

Les deux orifices de mesure 24, 25 sont préférentiellement fermés par une membrane fluidiquement étanche de manière à assurer le bon fonctionnement des microphones. En fait, la pression dans la chambre d'oscillation 2 se transmet aux microphones, via les deux orifices 24, 25, et au travers des membranes qui recouvrent ces deux orifices 24, 25. De préférence, la membrane a une épaisseur très fine au niveau des capteurs 24 et 25, typiquement de l'ordre de 50 à 500 µm environ ; ailleurs, son épaisseur peut être comprise entre 1 et 2 mm, voire plus.

En fait, en fonctionnement, le flux de gaz circule dans le sens des flèches (=>) représentées en Figure 1. Le flux de gaz, par exemple de l'oxygène ou de l'air médical, arrive par un canal d'entrée 4 est relié fluidiquement au premier orifice d'entrée 12 de la chambre de stabilisation 1 et pénètre dans ladite chambre de stabilisation 1, via ce premier orifice d'entrée 12.

Au sein de la chambre de stabilisation 1, le flux est soumis à une stabilisation par l'élément stabilisateur de flux 11, qui est de section se rapprochant de celle triangulaire avec sa base, c'est-à-dire sa face avant 1a, orientée en vis-à-vis du débouché du canal d'entrée 4, donc en face du premier orifice d'entrée 12. En fait, la section de l'élément stabilisateur de flux 11 est légèrement concave en se rapprochant de plus en plus de l'entrée 13 du conduit 3.

Le flux gazeux contourne donc l'élément stabilisateur de flux 11 en passant dans des passages 15 aménagés de part et d'autre de celui-ci. Les passages 15 sont en fait délimités par la surface externe de l'élément stabilisateur de flux 11 et par la paroi périphérique interne 14 de la chambre de stabilisation 1. En d'autres termes, l'élément stabilisateur de flux 11 est espacé de la paroi périphérique 14 de la chambre de stabilisation 1 de manière à créer des passages 15 pour le gaz autour dudit élément stabilisateur de flux 11.

Le flux gazeux ressort ensuite de la chambre de stabilisation 1 par le premier orifice de sortie 13 et est acheminé par le conduit de liaison 3 qui relie fluidiquement le premier orifice de sortie 13 de la chambre de stabilisation 1 au second orifice d'entrée 23 de la chambre d'oscillation 2.

Les premier et second orifices d'entrée 12, 23 et les premier et second orifices de sortie 13, 26 sont agencés de façon symétrique par rapport au plan de symétrie P.

Le gaz continue ensuite sa course dans la chambre d'oscillation 2 avant d'en ressortir par un conduit d'évacuation de gaz 27 qui est en communication fluidique avec le second orifice de sortie 26 de gaz de la chambre d'oscillation 2.

Le canal d'entrée 4 et le conduit d'évacuation de gaz 27 sont en communication avec le passage interne 33 du gaz du boitier 32 de la prise 31.

A partir d'un champ de vitesse symétrique en 2 dimensions, on crée un tourbillon dont la localisation, i.e. les zones Z1 et Z2, va osciller avec une fréquence proportionnelle à la valeur du débit du fluide qui y circule. En plaçant des microphones ou des organes/capteurs de mesure de pression en dehors du conduit du fluide, c'est-à-dire au-dessus des zones Z1, Z2 où se forment les tourbillons, i.e. les « vortex », on peut mesurer la présence ou non d'une dépression du gaz.

Le débitmètre 47 incorporé à la prise de distribution de gaz 31 de l'invention permet donc de déterminer, via une mesure de la fréquence d'oscillation des tourbillons de gaz, le débit de gaz qui y circule pour des valeurs de débits situées entre environ 0,5 et 15 L/min.

Des moyens de traitement de signal 49, encore appelés « électronique embarquée », « module de pilotage et de traitement de signal » ou « moyens de pilotage », telle une carte électronique à processeur, en particulier à microcontrôleur ou microprocesseur, mettant en œuvre un ou des algorithmes, sont reliés électriquement au débitmètre 47 de la prise de distribution de gaz 31, en particulier aux capteurs de pression ou microphones de manière à recueillir et exploiter les signaux de mesure de pression en extrayant la fréquence d'oscillation pour ensuite en déduire un débit de gaz.

Les moyens de traitement de signal 49 sont aussi reliés électriquement à au moins un module de télécommunication, comme détaillé ci-après, pour transmettre les valeurs de débit déterminées à partir des mesures de fréquence d'oscillation du gaz, comme expliqué ci-dessus.

La Figure 2 est une représentation tridimensionnelle du débitmètre 47 de la Figure 1 permettant de visualiser la localisation des orifices de mesure 24, 25 dans le plafond 28 de la chambre de reflux 2. Le débitmètre 47 est aménagé dans le boitier de prise 32 de la prise 31 de l'invention, comme expliqué ci-dessous.

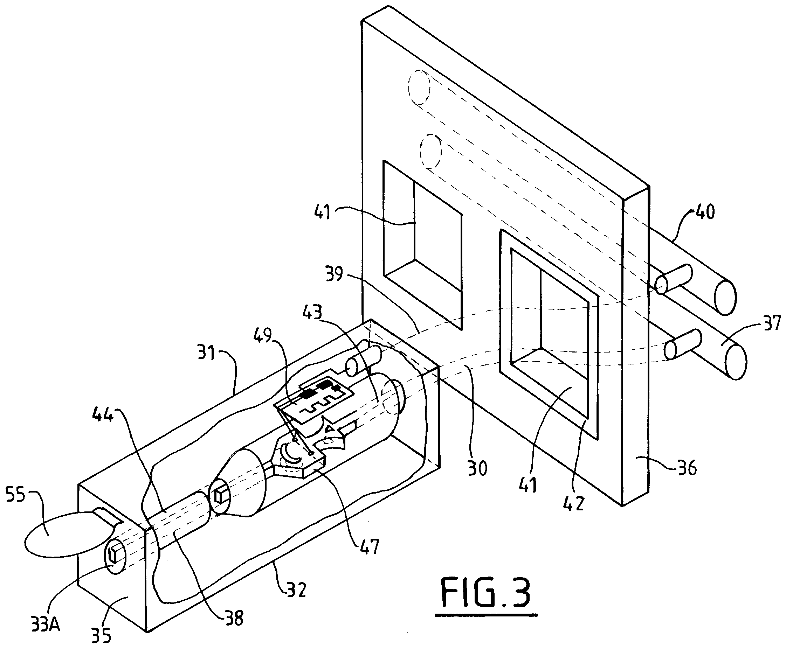

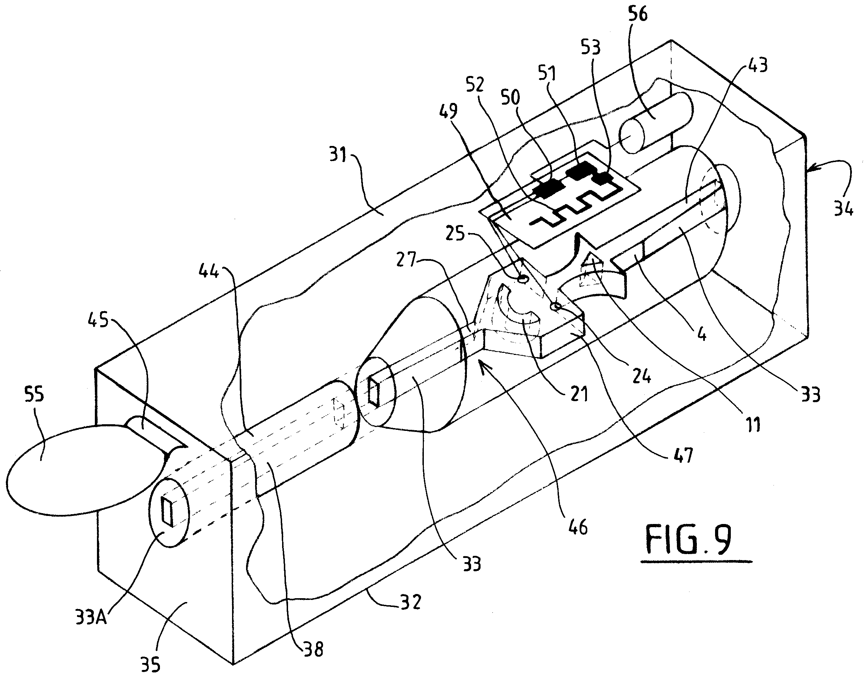

Les Figures 3 et 9 schématisent un premier mode de réalisation d'une prise de distribution de gaz 31 communicante selon la présente invention, c'est-à-dire une prise qui communique des informations, intégrant un débitmètre à oscillation fluidique 47 selon les Figures 1 et 2. La Figure 9 est une vue agrandie de la prise de distribution de gaz 31 de la Figure 3.

Comme détaillé en Figure 9, la prise de distribution de gaz 31 comprend un corps de prise 32, encore appelé armature ou boîtier de prise, incluant (au moins) un passage de gaz interne 33 permettant le passage d'un gaz au travers dudit corps de prise 32, dans le sens allant d'une face distale 34 à une face proximale 35 dudit corps de prise 32.

La face proximale 35 constitue une extrémité libre du corps de prise 32 et est adaptée et/ou configurée pour former un embout de connexion, encore appelé raccord de sortie, permettant un raccordement fluidique d'un connecteur externe, par exemple un connecteur équipant une conduite flexible de gaz destiné à relier fluidiquement l'embout de connexion de la prise de distribution de gaz 31 à un appareil médical, tel un ventilateur médical, utilisant le gaz distribué par la prise de distribution de gaz 31, ou encore à un dispositif de type débitlitre intégrant préférentiellement un détendeur de gaz.

Comme illustré en Figure 3, le corps de prise 32 peut être encastré dans un logement 41 aménagé dans une structure-support 36, tel un boitier ou analogue ou être fixé directement à une paroi ou analogue, en particulier une cloison, un mur ou un panneau d'équipement installé au sein d'un bâtiment hospitalier, abritant un réseau de canalisations de gaz 37, par exemple des canalisations d'oxygène, d'air médical et/ou de protoxyde d'azote alimentées par des sources de gaz situées dans ou à l'extérieur du bâtiment, tels des réservoirs de stockage, des cadres de bouteilles de gaz, ou une unité de production sur-site de gaz, tel une unité PSA (= *Pressure Swing Adsorption*) de production d'air médical par adsorption et variations de pression...

Par exemple, l'encastrement ou le montage du corps de prise 32 de la prise de distribution de gaz 31 dans la structure-support 36 du bâtiment hospitalier, tel un boitier de prise porté par un mur ou analogue, peut se faire en insérant le corps de prise 32 directement dans un logement de prise 41 complémentaire, telle une cavité, lequel logement de prise 41 est ménagé dans la structure-support 36 elle-même, ou, indirectement, via une structure d'accueil 42 spécifique, préalablement disposée dans le logement de prise 41, laquelle reçoit ensuite le corps de prise 32, comme illustré en Figure 3.

Comme montré en Figure 3, la portion amont 43 du passage de gaz interne 33 de la prise de distribution de gaz 31 est raccordé fluidiquement 30 au réseau de canalisations de gaz 37 de l'hôpital ou analogue.

De façon analogue, un ou des câbles de courant électrique 39 permettent de raccorder électriquement la prise de distribution de gaz 31 à une source de courant électrique, à savoir avantageusement au moins une batterie ou pile garantissant un fonctionnement pendant une ou plusieurs années, préférentiellement au moins 2 ans, encore plus préférentiellement au moins 3 ans, typiquement au moins 4 ans.

Toutefois, selon un autre mode de réalisation (non montré), le corps de prise 32 et la structure-support 36, tel un boitier de prise, peuvent aussi être formés d'une structure monobloc ou quasi-monobloc conçue pour venir se raccorder au réseau de canalisations 37 et au réseau électrique 40, et se fixer directement à une paroi ou analogue.

Le passage de gaz interne 33 du corps de prise 32 de la prise de distribution de gaz 31 comporte à son « extrémité libre », une partie ou portion aval 44 débouchant sur la face proximale 35 du boitier et comportant un orifice de sortie de gaz 33A débouchant sur ladite face proximale 35. L'extrémité libre du corps de prise 32 constitue un embout de connexion pour le raccordement d'un connecteur par exemple le connecteur de raccordement situé à une extrémité libre d'un conduit de gaz servant à alimenter un appareillage médical. En d'autres termes, l'embout de connexion fait office de connecteur mécanique et fluidique permettant un raccordement mécanique et fluidique d'un connecteur externe.

On prévoit en outre un système de contrôle du débit de gaz 38 comprenant des moyens de vanne agencés sur le passage de gaz interne 33 du corps de prise 32.

Les moyens de vanne sont du type valve à sens unique (i.e., *one-way valve* en anglais) et autorisent une circulation du gaz dans le passage de gaz interne du corps de prise 32, en direction de l'embout de connexion, lorsque le connecteur d'un conduit de gaz est raccordé audit embout de connexion. De préférence, les moyens de vanne comprennent un obturateur de vanne, tel un clapet mobile ou analogue, pivotant ou translatif, qui est normalement repoussé par un élément élastique, tel un ressort de rappel, vers sa position de fermeture, empêchant alors tout passage de gaz lorsqu'un connecteur ou analogue n'est pas raccordé à la prise 31. A l'inverse, l'obturateur de vanne est repoussé en position d'ouverture, de sorte de libérer le passage de gaz, lorsqu'un connecteur ou analogue est raccordé à la prise 31, tel un tuyau flexible ou un appareil médical.

La prise 31 de l'invention peut également comprendre un (ou plusieurs) élément d'étanchéité, telle une bague ou un joint d'étanchéité, et/ou un (ou plusieurs) élément de filtrage du gaz, par exemple un (ou plusieurs) filtre ou analogue, permettant d'éliminer les poussières, microorganismes ou autres impuretés susceptibles d'être véhiculées par le gaz.

De façon générale, le connecteur de gaz associé à l'appareillage à usage médical (non représenté) ou au tuyau flexible venant se fixer à la prise 31 est de type connu, par exemple ce peut être un connecteur à baïonnette enfichable dans la prise 31 tel que décrit par exemple dans le document EP-A-2055341. Ce connecteur est préférentiellement de type normalisé, par exemple selon la norme française AFNOR NF-S 90-116.

De préférence, l'embout de connexion porté par la portion amont 44 du corps 32 de prise présente, en face proximale 35, un bord périphérique ou profil particulier qui est conformé pour offrir un accouplement avec un raccord ou embout de connexion externe de forme spécifique, en fonction du type de gaz distribué par la prise 31, par exemple avec un connecteur d'un tuyau flexible associé à l'appareillage à usage médical ou analogue, par exemple un appareillage qui utilise un gaz déterminé à des fins thérapeutiques, un instrument chirurgical ou des appareils de même type, ou bien un appareillage technique hospitalier, c'est-à-dire un appareillage de diagnostic, de contrôle d'appareillages médicaux ou pour l'entraînement d'instruments ou d'appareils médicaux.

Par ailleurs, comme déjà mentionné et détaillé en Figure 9, le passage de gaz interne 33 comporte une partie ou portion intermédiaire 46 située entre la portion amont 43 et la portion aval 44, qui comprend un débitmètre à oscillateur fluidique 47, tel celui représenté sur les Figures 1 et 2, agencé de manière à être en communication fluidique avec le passage de gaz interne 33. La géométrie du circuit de gaz interne du débitmètre à oscillateur fluidique 47 est de préférence choisie pour que des oscillations du gaz soient déclenchées et soutenues pour des débit de gaz allant jusqu'à 100 L/min, lorsque le gaz considéré est de l'oxygène, de l'air médical ou leurs mélanges, à une température comprise entre 0°C et 40 °C, de préférence entre 0°C et 30°C, pour une pression du gaz comprise entre 4 et 10 bar relatif.

Comme visible en Figure 9, la prise de distribution de gaz 31 communicante de l'invention comporte par ailleurs des moyens de traitement de signal 49, c'est-à-dire une (ou plusieurs) carte électronique, encore appelée « électronique embarquée », comprenant processeur 50, tel un microcontrôleur ou microprocesseur, et au moins un module de communication comprenant au moins un modem radio 51, éventuellement intégré au processeur 50, associé à au moins une antenne radio 52, et éventuellement à un réseau d'adaptation d'impédance 53, de manière à permettre à la prise 31 de communiquer à distance.

On prévoit en outre une source d'énergie électrique 56, de préférence une pile, une batterie ou un connecteur électrique et mécanique permettant de relier électriquement les moyens de traitement de signal 49, typiquement une carte électronique, au réseau de courant électrique 40, comme expliqué ci-avant, de manière à alimenter en courant électrique, les moyens de traitement de signal 49 ou d'autres éléments de la prise, comme le (ou les) moyen 48 de détection des oscillations fluidiques du débitmètre à oscillateur fluidique 47, typiquement des microphones.

De préférence, les moyens de traitement de signal 49, c'est-à-dire l'électronique embarquée ou analogue, comprennent au moins un premier module de télécommunication comprenant un premier modem radio 51, une première antenne radio 52 et un premier réseau d'adaptation d'impédance 53, configuré pour communiquer des données selon une technologie de type LoRa, Sigfox, LTE-M Cat 0, EC-GSM ou NB-loT.

Pour ce faire, le premier module de télécommunication présente avantageusement une sensibilité inférieure ou égale à -110 dBm et une valeur d'affaiblissement de couplage ou valeur « MCL » supérieure ou égale à 130 dB avec les stations de base du réseau de communication considéré de manière à pouvoir réaliser des transmissions de données sur de longues distances, typiquement de 100 m à 30 km environ, selon l'environnement de propagation des signaux considérés, i.e. milieu urbain ou dégagé.

Ainsi, le premier modem radio 51 peut être configuré pour opérer au moins une modulation
- du type à étalement de spectre, de préférence au moins une modulation de type LoRa, éventuellement associée à un protocole d'accès de type LoRaWAN ; ou
- du type à bande ultra-étroite, de préférence au moins une modulation de type Sigfox, éventuellement associée à un protocole d'accès Sigfox.

Alternativement, le premier modem radio 51 peut être configuré pour opérer au moins une modulation et un protocole ou une pile de protocoles associés, sur au moins à un réseau de type EC-GSM, NB-loT ou LTE-M.

De préférence, le premier modem radio 51 est configuré pour opérer selon un débit de transmission compris entre 100 bits par seconde, comme dans le cas d'un réseau Sigfox, et 1 Mégabit par seconde, comme dans le cas d'un réseau LTE-M Cat0, c'est-à-dire dans tout ou partie de cette plage de débit de transmission.

Par ailleurs, l'électronique embarquée 49 peut aussi comprendre un second module de télécommunication (non représenté) comprenant un deuxième modem radio, une deuxième antenne radio et un deuxième réseau d'adaptation d'impédance configuré pour communiquer des données selon une seconde technologie de type Bluetooth Low Energy, Bluetooth ou NFC. De ce fait, la première antenne radio 52 peut être une antenne unique commune aux deux modules de télécommunication ou, selon le cas, être dissociée en deux antennes distinctes spécifiques chacune d'une technologie radio, donc associées chacune à l'un des modules de télécommunication.

De préférence, l'électronique 49 embarquée comprend aussi une ou plusieurs mémoires électroniques, des moyens analogiques ou numériques de conditionnement des signaux générés par le moyen 48 de détection des oscillations fluidiques du débitmètre à oscillateur fluidique 47, typiquement des microphones, et une horloge temps-réel.

Optionnellement, elle peut également comprendre un interrupteur de marche/arrêt, un témoin de fonctionnement, par exemple une diode électroluminescente, et/ou un port de programmation du processeur 50, typiquement un microcontrôleur.

Dans tous les cas, l'électronique embarquée 49 opère une quantification des oscillations fluidiques et l'estimation du débit de gaz traversant le circuit de gaz 33, à partir des valeurs de fréquence transmises par le débitmètre à oscillateur fluidique 47.

L'électronique embarquée 49 réalise éventuellement un stockage de ces estimations de débit en mémoire, par exemple au sein d'une mémoire flash, et surtout leur transmission sans fil par radio, par exemple à un serveur distant, via le (ou les) module de télécommunication.

L'électronique embarquée 49 peut également réaliser le stockage en mémoire d'une information de configuration, par exemple, une information de localisation au sein d'un bâtiment ou un identifiant d'appareillage médical connecté à la prise 31 de l'invention, ou de personnel ou de patient utilisant un tel appareillage médical, qui est transmise par radio.

La prise de distribution de gaz 31 de l'invention peut également comprendre un élément d'obturation mobile 55, par exemple un capot ou clapet monté sur charnière 45, permettant de recouvrir au moins une partie de la face proximale 35 du corps de prise 32, en particulier l'orifice de sortie 33A du passage interne de gaz 33 pour empêcher l'entrée de poussières ou analogue.

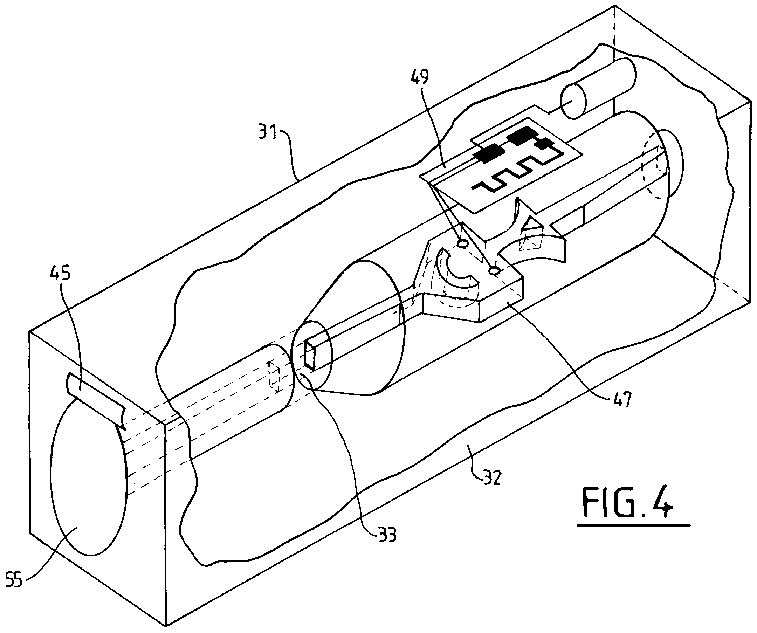

Avantageusement, la ou les antennes 52 et leurs éventuels réseaux d'adaptation d'impédance associés 53 sont choisis de sorte que les « qualités radio » de la prise 31 soient au moins meilleures lorsque l'élément d'obturation mobile 55 est en position « ouverte » (cf. Figures 3 et 9) que lorsqu'il est en position « fermée » (cf. Figure 4). Par "qualité radio", on entend par exemple le gain d'antenne mesuré sur la prise intégrée 31 et le caractère omnidirectionnel du diagramme de rayonnement radio dans un plan ou dans l'espace, constaté sur la prise 31 intégrée.

De préférence, le corps de prise 32 est réalisé en matériau perméable aux ondes radio, par exemple le matériau peut être un matériau plastique, tel que du polycarbonate ou analogue.

La Figure 4 est identique à la Figure 9, à l'exception du fait qu'elle schématise la prise de distribution de gaz 31 avec l'élément d'obturation mobile 55 en position « fermée », alors que sur la Figure 9, il est en position « ouverte ».

Lorsqu'il est en position « fermée » et qu'il obture l'orifice de sortie du gaz 33A, l'élément d'obturation mobile 55 empêche les dépôts de poussières ou autres au niveau de l'extrémité de sortie du passage interne de gaz 33 débouchant au travers de l'orifice de sortie 33A, ce qui évite que ceux-ci ne se retrouvent dans les canalisations ou autres que l'on vient raccorder à la prise de distribution de gaz 31.

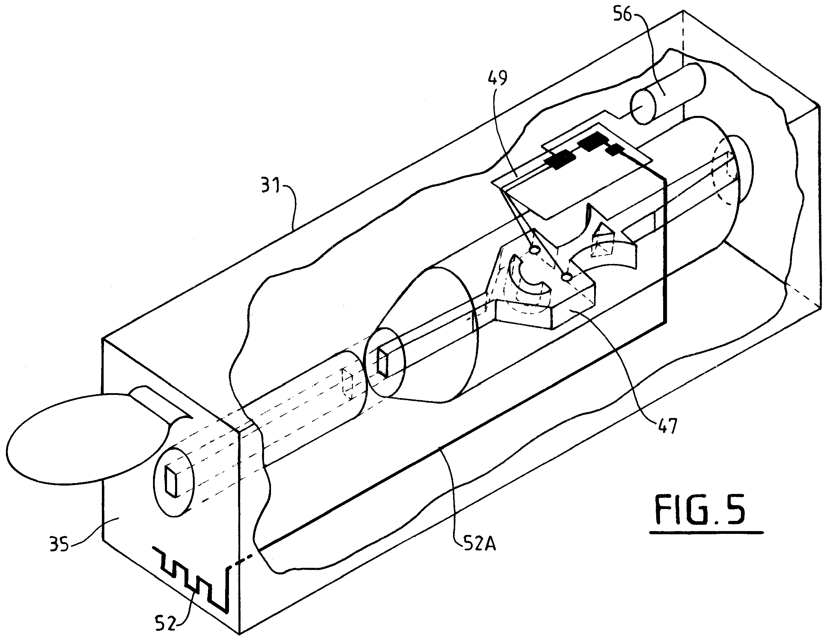

La Figure 5 illustre un second mode de réalisation de la prise de distribution de gaz 31, dans lequel une (ou plusieurs) antenne 52 est agencée sur la face proximale 35 de la prise 31 et ce, que ce soit à l'intérieur ou à l'extérieur du corps de prise 32, ou en étant incorporé à la structure-même du corps de prise 32. L'antenne 52 est reliée électriquement à l'électronique embarquée 49, telle une carte électronique ou analogue, par une connectique 52A appropriée.

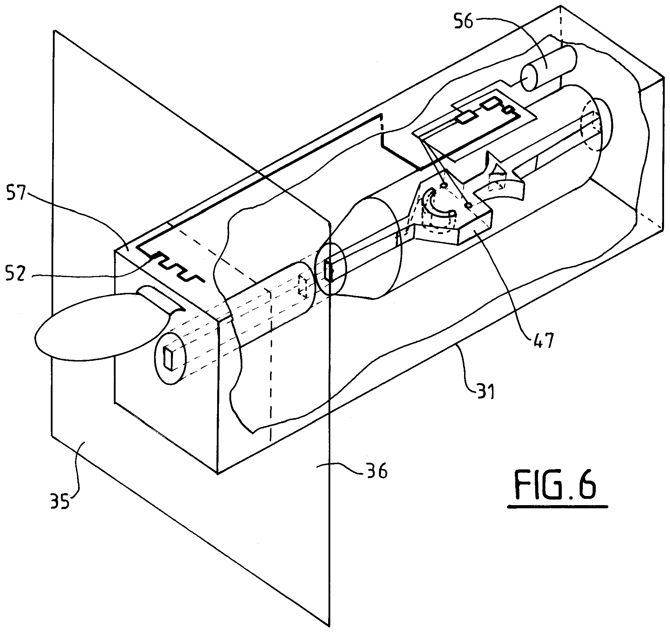

La Figure 6 illustre un troisième mode de réalisation de la prise de distribution de gaz 31, dans lequel une (ou plusieurs) antenne 52 est agencée dans une portion externe 57 de la prise 31 qui est elle-même encastrée dans une structure-support 36 de type cloison ou mur. La portion externe 57 correspond à la partie d'extrémité de la prise 31 émergeant de la structure-support 36, lorsque la prise 1 y est encastrée, c'est-à-dire à la partie d'extrémité de la prise 31 faisant saillie à l'extérieur de la structure-support 36, lorsque la prise 31 y est enfichée.

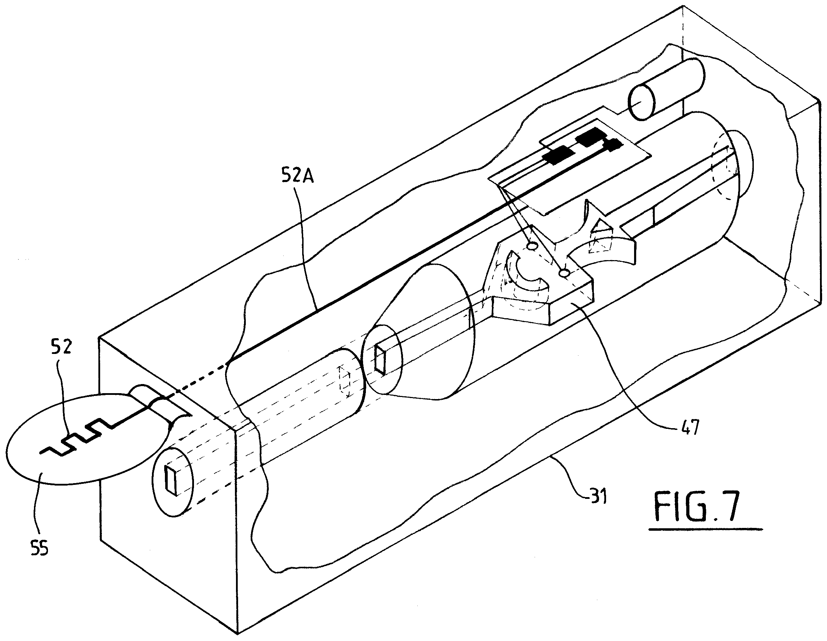

La Figure 7 illustre un quatrième mode de réalisation de la prise de distribution de gaz 31, dans lequel une (ou plusieurs) antenne 52 est agencée sur l'élément d'obturation mobile 55, c'est-à-dire à sa surface ou en étant intégré dans le corps lui-même de l'élément d'obturation mobile 55. L'élément d'obturation mobile 55 est représenté ici en position « ouverte ». Là encore, l'antenne 52 est reliée électriquement à l'électronique embarquée 49, via une connectique 52A appropriée, tel un (ou des) câble électrique ou analogue.

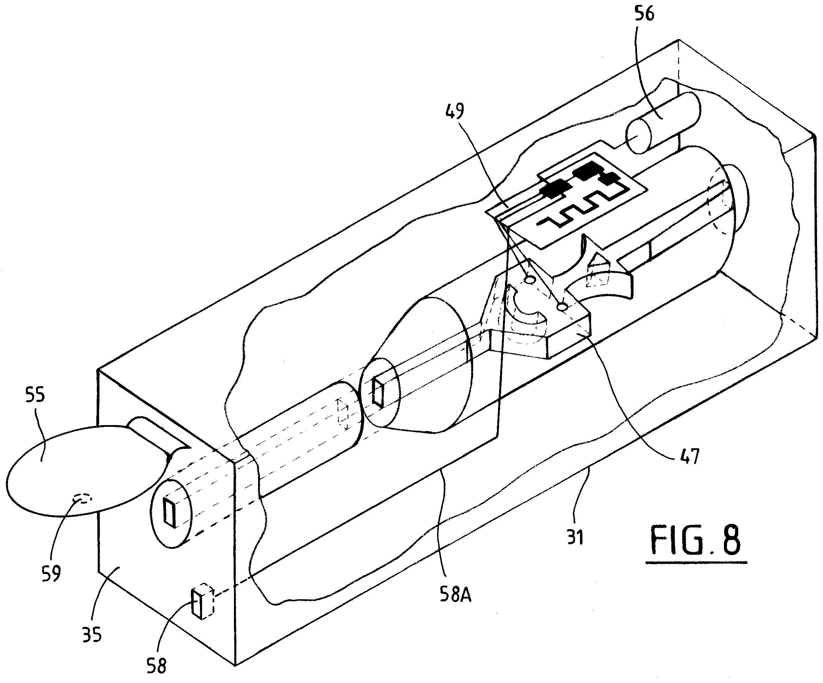

La Figure 8 représente une prise de distribution de gaz 31 selon l'invention identique à celle de la Figure 9, à l'exception de l'agencement supplémentaire d'un capteur de position 58 optionnel sur la prise 31 permettant de déterminer la position « ouverte » ou « fermée » de l'élément d'obturation mobile 55.

Le capteur de position 58 est agencé ici au niveau de la face avant 35 de la prise 31.

Le capteur de position 58 peut être par exemple un capteur capacitif ou un contacteur REED réagissant au voisinage d'un élément de détection 59, tel un élément métallique ou un aimant, agencé sur l'élément d'obturation mobile 55. En d'autres termes, le capteur de position 58 coopère avec un élément de détection 59 porté par l'obturateur 55 pour déterminer la position ouverte ou fermée dudit obturateur 55.

Le capteur de position 58 est relié électriquement à l'électronique embarquée 49, via une connectique 58A adaptée, tel un (ou des) câble électrique ou analogue, pour lui transmettre un signal de détection (ou de non détection) de l'élément de détection 59.

Utiliser un tel capteur de position 58 est avantageux car la mise en marche ou, à l'inverse, l'arrêt de l'électronique embarquée 49 peut être conditionnée à la détection de la position « ouverte » ou « fermée » de l'élément d'obturation mobile 55 par le capteur de position 58. De plus, la détection de la position « fermée » de l'élément d'obturation mobile 55 peut également entraîner le déclenchement d'une logique de basse consommation dans l'électronique embarquée 49, par exemple une mise en veille temporaire des composants, ou un réveil périodique des composants moins fréquent que lorsque l'élément d'obturation mobile 5 est en position « ouverte », ou encore des transmissions radio moins fréquentes que lorsque l'élément d'obturation mobile 55 est en position « ouverte ».

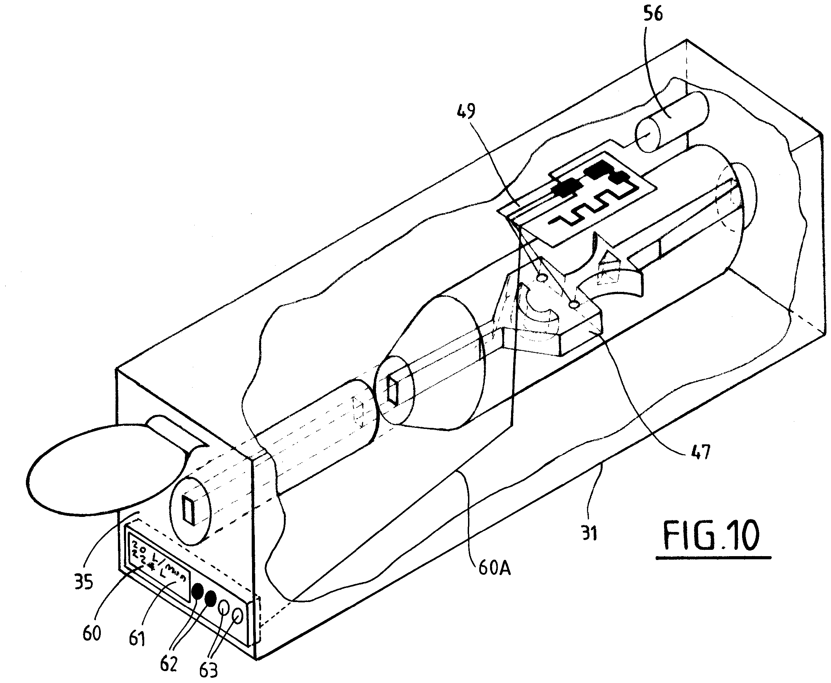

Enfin, la Figure 10 schématise un mode de réalisation de la prise de distribution de gaz 31 selon l'invention, dans lequel la prise 31 est identique à celle des Figures 3 et 9, à l'exception du fait qu'elle intègre une interface utilisateur 60 agencée par exemple en face proximale 35, qui est en liaison électrique 60A, via un (ou des) câble ou analogue, avec l'électronique embarquée, c'est-à-dire les moyens de traitement de signal 49.

Cette interface utilisateur 60 peut par exemple comporter :
- un afficheur 61 de données, par exemple un dispositif de type « e-paper », ou un afficheur alphanumérique matriciel ou à segments, destiné à afficher par exemple le débit instantané estimé grâce à l'oscillateur fluidique 47, la valeur intégrée par l'électronique 49 sur une période de temps donnée du débit instantané, des icônes indiquant l'état de fonctionnement ou de configuration de la prise... ou d'autres données ou informations, et/ou
- un ou plusieurs témoins 62 de fonctionnement ou de retour d'état de l'électronique 49, par exemple des diodes électroluminescentes, et/ou
- un ou plusieurs boutons, touches ou interrupteurs 63, permettant par exemple de commander la marche ou l'arrêt de tout ou partie de l'électronique 49 ou de l'interface 60, la remise à zéro de la valeur intégrée du débit instantané, un changement de configuration de la prise, un envoi de données par radio, un appairage avec un dispositif tiers à travers une liaison radio Bluetooth ou Bluetooth Low Energy, ou de toute autre fonction.

D'une façon générale, la prise de distribution de gaz 31 communicante selon la présente invention, qui est équipée d'un débitmètre à oscillation fluidique et de moyens de télécommunication, est conçue pour être fixée sur ou encastrée à une paroi-support, donc elle est particulièrement bien adaptée à une utilisation pour distribuer un ou des gaz médicaux dans un bâtiment hospitalier ou analogue, voire dans un véhicule d'urgence mobile de type ambulance, SAMU...

## Revendications

1. Prise de distribution de gaz (31) communicante comprenant :
- un corps de prise (32) traversé par au moins un passage de gaz interne (33) permettant le passage d'un gaz au travers dudit corps de prise (32),
- un système de contrôle du débit de gaz (38) agencé sur le passage de gaz interne (33) du corps de prise (32),
- un débitmètre à oscillation fluidique (47) agencé sur le passage de gaz interne (33) et comprenant deux microphones conçus et agencés pour mesurer une fréquence d'oscillation de tourbillons de gaz, ledit débitmètre à oscillation fluidique (47) étant configuré pour fournir ledit au moins un signal de fréquence d'oscillation de tourbillons de gaz à des moyens de traitement de signal (49),
- des moyens de traitement de signal (49) configurés pour récupérer et traiter le ou les signaux de mesure délivrés par le débitmètre à oscillation fluidique (47), les moyens de traitement de signal (49) étant configurés pour transformer au moins une valeur de fréquence délivrée par le débitmètre à oscillation fluidique (47) en au moins une valeur de débit,
- au moins un module de télécommunication (51, 52) comprenant au moins un modem radio (51) associé à au moins une antenne émettrice/réceptrice (52), configuré pour transmettre sans fil un ou des signaux de mesure provenant des moyens de traitement de signal (49).

2. Prise de distribution de gaz selon la revendication précédente, **caractérisée en ce que** :
- le passage de gaz interne (33) comprend une extrémité libre formant embout de connexion pour le raccordement d'un connecteur,
- le système de contrôle du débit de gaz (38) comprend des moyens à vanne autorisant une circulation du gaz dans le passage de gaz interne (33) du corps de prise (32), en direction d'un orifice de sortie de gaz (33A) porté par l'extrémité libre formant embout de connexion, lorsqu'un connecteur est raccordé à ladite l'extrémité libre formant embout de connexion,
- le débitmètre à oscillation fluidique (47) est agencé dans le corps de prise (32),
- les moyens de traitement de signal (49) sont reliés électriquement au débitmètre à oscillation fluidique (47), et/ou
- ledit au moins un module de télécommunication (51, 52) est relié électriquement aux moyens de traitement de signal (49) et/ou configuré pour transmettre sans fil un ou des signaux de mesure provenant des moyens de traitement de signal (49) à au moins un réseau de communication.

3. Prise de distribution de gaz selon l'une des revendications précédentes, **caractérisée en ce que** ledit au moins un module de télécommunication (51, 52) est configuré pour communiquer des données selon une technologie de type LoRa, Sigfox, LTE-M Cat 0, EC-GSM ou NB-loT.

4. Prise de distribution de gaz selon l'une des revendications précédentes, **caractérisée en ce qu'**elle comprend en outre une source de courant électrique (56) alimentant les moyens de traitement de signal (49) et/ou le module de télécommunication (51, 52).

5. Prise de distribution de gaz selon l'une des revendications précédentes, **caractérisée en ce que** le module de télécommunication (51, 52) est configuré pour transmettre sans fil au moins une valeur de débit provenant des moyens de traitement de signal (49) à au moins un réseau de communication.

6. Prise de distribution de gaz selon l'une des revendications précédentes, **caractérisée en ce qu'**elle comprend un élément d'obturation mobile (55) permettant de recouvrir au moins une partie de la face proximale (35) du corps de prise (32) comportant l'orifice de sortie (33A) du passage interne de gaz (33), une antenne (52) étant agencée sur l'élément d'obturation mobile (55).

7. Prise de distribution de gaz selon l'une des revendications précédentes, **caractérisée en ce qu'**elle comprend une interface utilisateur (60) reliée électriquement aux moyens de traitement de signal (47).

8. Prise de distribution de gaz selon l'une des revendications précédentes, **caractérisée en ce qu'**au moins un premier module de télécommunication présente une sensibilité inférieure ou égale à -110 dBm et une valeur d'affaiblissement de couplage ou valeur « MCL » supérieure ou égale à 130 dB.

9. Prise de distribution de gaz selon l'une des revendications précédentes, **caractérisée en ce que** les moyens de traitement de signal (49) sont reliés électriquement aux microphones du débitmètre à oscillation fluidique (47).

10. Prise de distribution de gaz selon l'une des revendications précédentes, **caractérisée en ce que** les moyens de traitement de signal (49), reliés électriquement aux microphones, sont configurés pour déduire de la fréquence d'oscillation de tourbillons de gaz mesurée par lesdits microphones, un débit de gaz compris entre environ 0,5 et 15 sL/min.

11. Prise de distribution de gaz selon l'une des revendications précédentes, **caractérisée en ce que** la source de courant électrique (56) comprend au moins une pile ou batterie.

12. Prise de distribution de gaz selon l'une des revendications précédentes, **caractérisée en ce que** la source de courant électrique (56) comprend au moins une pile ou batterie assurant une autonomie électrique d'au moins 2 ans.

13. Utilisation d'une prise de distribution de gaz (31) communicante selon l'une des revendications précédentes pour distribuer un gaz au sein d'un bâtiment hospitalier, en particulier de l'oxygène, de l'air ou du protoxyde d'azote (N₂O).

14. Bâtiment hospitalier comprenant au moins une prise de distribution de gaz communicante selon l'une des revendications 1 à 12 pour distribuer un gaz au sein dudit bâtiment hospitalier, en particulier de l'oxygène, de l'air ou du protoxyde d'azote (N₂O), en particulier ladite au moins une prise de distribution de gaz est agencée sur un réseau de canalisations de gaz parcourant le bâtiment hospitalier.

## Patentansprüche

1. Kommunizierender Gasausgabeanschluss (31), umfassend:
- einen Anschlusskörper (32), der von mindestens einem internen Gasdurchlass (33) durchquert wird, der den Durchlass eines Gases durch den Anschlusskörper (32) ermöglicht,
- ein System zur Steuerung des Gasdurchsatzes (38), das an dem internen Gasdurchlass (33) des Anschlusskörpers (32) angeordnet ist,
- einen Durchflussmesser nach dem Schwingstrahlprinzip (47), der auf dem internen Gasdurchlass (33) angeordnet ist und zwei Mikrofone umfasst, die zum Messen einer Gaswirbeloszillationsfrequenz ausgelegt und angeordnet sind, wobei der Durchflussmesser nach dem Schwingstrahlprinzip (47) konfiguriert ist, um das mindestens eine Gaswirbeloszillationsfrequenzsignal an Signalverarbeitungsmittel (49) bereitzustellen,
- Signalverarbeitungsmittel (49), die konfiguriert sind, um das oder die von dem Durchflussmesser nach dem Schwingstrahlprinzip (47) gelieferte(n) Messsignal(e) abzurufen und zu verarbeiten, wobei die Signalverarbeitungsmittel (49) konfiguriert sind, um mindestens einen Frequenzwert, der von dem Durchflussmesser nach dem Schwingstrahlprinzip (47) geliefert wird, in mindestens einen Durchsatzwert umzuwandeln,
- mindestens ein Telekommunikationsmodul (51, 52), das mindestens ein Funkmodem (51) umfasst, das mindestens einer Sender-/Empfängerantenne (52) zugeordnet ist, das konfiguriert ist, um drahtlos ein oder mehrere Messsignale von den Signalverarbeitungsmitteln (49) zu übertragen.

2. Gasausgabeanschluss nach dem vorstehenden Anspruch, **dadurch gekennzeichnet, dass**:
- der interne Gasdurchlass (33) ein freies Ende umfasst, das ein Verbindungsanschlussstück zum Anschluss eines Verbinders bildet,
- das System zur Steuerung des Gasdurchsatzes (38) Ventilmittel umfasst, die eine Zirkulation des Gases durch den internen Gasdurchlass (33) des Anschlusskörpers (32) in Richtung einer Gasausgangsöffnung (33A) erlauben, die von dem freien Ende getragen wird, das ein Verbindungsanschlussstück bildet, wenn ein Verbinder an das freie Ende, das ein Verbindungsanschlussstück bildet, angeschlossen ist,
- der Durchflussmesser nach dem Schwingstrahlprinzip (47) in dem Anschlusskörper (32) angeordnet ist,
- die Signalverarbeitungsmittel (49) elektrisch mit dem Durchflussmesser nach dem Schwingstrahlprinzip (47) verbunden sind, und/oder
- das mindestens eine Telekommunikationsmodul (51, 52) elektrisch mit den Signalverarbeitungsmitteln (49) verbunden ist und/oder konfiguriert ist, um drahtlos ein oder mehrere Messsignale von den Signalverarbeitungsmitteln (49) an mindestens ein Kommunikationsnetzwerk zu übertragen.

3. Gasausgabeanschluss nach einem der vorstehenden Ansprüche, **dadurch gekennzeichnet, dass** das mindestens eine Telekommunikationsmodul (51, 52) konfiguriert ist, um Daten gemäß einer Technologie vom Typ LoRa, Sigfox, LTE-M Cat 0, EC-GSM oder NB-IoT zu kommunizieren.

4. Gasausgabeanschluss nach einem der vorstehenden Ansprüche, **dadurch gekennzeichnet, dass** er weiter eine elektrische Stromquelle (56) umfasst, welche die Signalverarbeitungsmittel (49) und/oder das Telekommunikationsmodul (51, 52) versorgt.

5. Gasausgabeanschluss nach einem der vorstehenden Ansprüche, **dadurch gekennzeichnet, dass** das Telekommunikationsmodul (51, 52) konfiguriert ist, um drahtlos mindestens einen Durchsatzwert von den Signalverarbeitungsmitteln (49) an mindestens ein Kommunikationsnetzwerk zu übertragen.

6. Gasausgabeanschluss nach einem der vorstehenden Ansprüche, **dadurch gekennzeichnet, dass** er ein mobiles Verschlusselement (55) umfasst, das es ermöglicht, mindestens einen Teil der proximalen Seite (35) des Anschlusskörpers (32) mit der Ausgangsöffnung (33A) des internen Gasdurchlasses (33) abzudecken, wobei eine Antenne (52) auf dem mobilen Verschlusselement (55) angeordnet ist.

7. Gasausgabeanschluss nach einem der vorstehenden Ansprüche, **dadurch gekennzeichnet, dass** er eine Benutzerschnittstelle (60) umfasst, die elektrisch mit den Signalverarbeitungsmitteln (47) verbunden ist.

8. Gasausgabeanschluss nach einem der vorstehenden Ansprüche, **dadurch gekennzeichnet, dass** mindestens ein erstes Telekommunikationsmodul eine Empfindlichkeit kleiner oder gleich -110 dBm und einen Kopplungsdämpfungswert oder "MCL"-Wert größer oder gleich 130 dB aufweist.

9. Gasausgabeanschluss nach einem der vorstehenden Ansprüche, **dadurch gekennzeichnet, dass** die Signalverarbeitungsmittel (49) elektrisch mit den Mikrofonen des Durchflussmessers nach dem Schwingstrahlprinzip (47) verbunden sind.

10. Gasausgabeanschluss nach einem der vorstehenden Ansprüche, **dadurch gekennzeichnet, dass** die Signalverarbeitungsmittel (49), die elektrisch mit den Mikrofonen verbunden sind, konfiguriert sind, um aus der Gaswirbeloszillationsfrequenz, die von den Mikrofonen gemessen wird, einen Gasdurchsatz zwischen etwa 0,5 und 15 sL/min abzuleiten.

11. Gasausgabeanschluss nach einem der vorstehenden Ansprüche, **dadurch gekennzeichnet, dass** die elektrische Stromquelle (56) mindestens eine Zelle oder Batterie umfasst.

12. Gasausgabeanschluss nach einem der vorstehenden Ansprüche, **dadurch gekennzeichnet, dass** die elektrische Stromquelle (56) mindestens eine Zelle oder Batterie umfasst, die eine elektrische Autonomie von mindestens 2 Jahren gewährleistet.

13. Verwendung eines kommunizierenden Gasausgabeanschlusses (31) nach einem der vorstehenden Ansprüche, um ein Gas, insbesondere Sauerstoff, Luft oder Lachgas (N₂O), innerhalb eines Krankenhausgebäudes auszugeben.

14. Krankenhausgebäude, das mindestens einen kommunizierenden Gasausgabeanschluss nach einem der Ansprüche 1 bis 12 zum Ausgeben eines Gases, insbesondere von Sauerstoff, Luft oder Lachgas (N₂O), innerhalb des Krankenhausgebäudes umfasst, wobei insbesondere der mindestens eine Gasausgabeanschluss auf einem Netz von Gasleitungen angeordnet ist, die durch das Krankenhausgebäude verlaufen.

## Claims

1. Communicating gas distribution outlet (31) comprising:
- an outlet body (32) passed through by at least one inner gas passage (33) making it possible for the passage of a gas through said outlet body (32),
- a system for controlling the gas flow (38) arranged on the inner gas passage (33) of the outlet body (32),
- a fluidic oscillation flow sensor (47) arranged on the inner gas passage (33) and comprising two microphones designed and arranged to measure an oscillation frequency of gas tourbillons, said fluidic oscillation flow sensor (47) being configured to provide said at least one oscillation frequency signal of gas tourbillons to the signal processing means (49),
- signal processing means (49) configured to recover and process the measuring signal(s) delivered by the fluidic oscillation flow sensor (47), the signal processing means (49) being configured to transform at least one frequency value delivered by the fluidic oscillation flow sensor (47) into at least one flow value,
- at least one telecommunication module (51, 52) comprising at least one radio modem (51) associated with at least one emitting/receiving antenna (52), configured to wirelessly transmit one or more measuring signals coming from the signal processing means (49).

2. Gas distribution outlet according to the preceding claim, **characterised in that**:
- the inner gas passage (33) comprises a free end forming a connecting piece to connect a connector,
- the system for controlling the gas flow (38) comprises valve means enabling a gas circulation in the inner gas passage (33) of the outlet body (32), in the direction of a gas outlet orifice (33A) supported by the free end forming a connecting piece, when a connector is connected to said free end forming a connecting piece,
- the fluidic oscillation flow sensor (47) is arranged in the outlet body (32),
- the signal processing means (49) are electrically connected to the fluidic oscillation flow sensor (47), and/or
- said at least one telecommunication module (51, 52) is electrically connected to the signal processing means (49) and/or configured to wirelessly transmit one or more measuring signals coming from the signal processing means (49) to at least one communication network.

3. Gas distribution outlet according to one of the preceding claims, **characterised in that** said at least one telecommunication module (51, 52) is configured to communicate data according to an LoRa, Sigfox, LTE-M, Cat 0, EC-GSM or NB-IoT type technology.

4. Gas distribution outlet according to one of the preceding claims, **characterised in that** it further comprises an electric current source (56) supplying the signal processing means (49) and/or the telecommunication module (51, 52).

5. Gas distribution outlet according to one of the preceding claims, **characterised in that** the telecommunication module (51, 52) is configured to wirelessly transmit at least one flow value coming from the signal processing means (49) to at least one communication network.

6. Gas distribution outlet, according to one of the preceding claims, **characterised in that** it comprises a mobile blocking element (55) making it possible to cover at least one portion of the proximal face (35) of the outlet body (32) comprising the outlet orifice (33A) of the inner gas passage (33), an antenna (52) being arranged on the mobile blocking element (55).

7. Gas distribution outlet according to one of the preceding claims, **characterised in that** it comprises a user interface (60) electrically connected to the signal processing means (47).

8. Gas distribution outlet according to one of the preceding claims, **characterised in that** at least one first telecommunication module has a sensitivity less than or equal to -110dBm and an attenuation coupling value or "MCL" value greater than or equal to 130dB.

9. Gas distribution outlet according to one of the preceding claims, **characterised in that** the signal processing means (49) are electrically connected to the microphones of the fluidic oscillation flow sensor (47).

10. Gas distribution outlet according to one of the preceding claims, **characterised in that** the signal processing means (49), electrically connected to the microphones, are configured to deduce the oscillation frequency of gas tourbillons measured by said microphones, a gas flow of between around 0.5 and 15 sL/min.

11. Gas distribution outlet according to one of the preceding claims, **characterised in that** the electric current source (56) comprises at least one cell or battery.

12. Gas distribution outlet according to one of the preceding claims, **characterised in that** the electric current source (56) comprises at least one cell or battery ensuring an electric autonomy of at least 2 years.

13. Use of a communicating gas distribution outlet (31) according to one of the preceding claims to distribute a gas within a hospital building, in particular, oxygen, air or nitrogen protoxide (N₂O).

14. Hospital building comprising at least one communicating gas distribution outlet according to one of claims 1 to 12, to distribute a gas within said hospital building, in particular, oxygen, air or nitrogen protoxide (N₂O), in particular said at least one gas distribution outlet is arranged on the gas pipeline network covering the hospital building.
